# EUROPEAN PATENT SPECIFICATION

(11) **EP 3 651 749 B1**
(45) Date of publication and mention of the grant of the patent: **04.09.2024**
(21) Application number: 17743406.5
(22) Date of filing: 12.07.2017
(51) Int. Cl.: A61K 31/04, A61K 31/202, A61K 31/375, A61K 31/7068, A61K 31/7072, A61P 25/28

(54) **METHOD FOR TREATING TRAUMATIC BRAIN INJURY**
VERFAHREN ZUR BEHANDLUNG TRAUMATISCHER HIRNLÄSIONEN
MÉTHODE DE TRAITEMENT DE LÉSION CÉRÉBRALE TRAUMATIQUE

(43) Date of publication of application: 20.05.2020
(73) Proprietor: N.V. Nutricia, 2712 HM Zoetermeer (NL)
(72) Inventor: DE WILDE, Mattheus Cornelis, 3584 CT Utrecht (NL); HAGEMAN, Robert Johan Joseph, 3584 CT Utrecht (NL)
(74) Representative: Nederlandsch Octrooibureau
(86) International application number: PCT/NL2017/050467
(87) International publication number: WO 2019/013615

(56) References cited:
- WO-A1-2012/125034
- WO-A1-2015/115885
- CN-A- 103 371 993
- US-A1- 2014 256 813

## Description

### Field of the invention

The present invention is in the field of medical nutrition and more particularly relates to nutritional compositions for use in the treatment of and recovery from traumatic brain injury and the symptoms associated with it.

### Background description

Traumatic brain injury (TBI) is a form of non-degenerative acquired brain injury. TBI is a serious medical condition that may occur after the brain is subjected to a significant external physical impact. TBI is the leading cause of disability and death in people under 45 with approximately 10 million new cases each year worldwide.

According to the diagnostic criteria detailed in the "Diagnostic and Statistical Manual of Mental Disorders (DSM-5) TBI is associated with the unique combination of one or more of the following characteristics: changes in levels of consciousness; memory disturbances; confusion associated with deficits in orientation; neurological signs, such as brain injury observable on neuroimaging, new onset or worsening of seizure disorder, visual field deficits and hemiparesis. While some symptoms may appear immediately after the injury, others may evolve over time consistent with anatomical changes in the neural substrates following the injury.

The primary phase of TBI describes immediate brain tissue damage from contusions or oxygen deprivation caused by global mass effect. The primary injury in TBI can only be reduced through improved prevention. Secondary injury starts immediately after trauma and underlies the functional deficits associated with TBI. It occurs later via such mechanisms as reperfusion injury, delayed cortical edema, blood-brain barrier (BBB) breakdown, glutaminergic overexcitation and local electrolyte imbalance. These disturbances themselves result in reactive oxygen species (ROS)-mediated neurodegeneration through calcium release, glutamate toxicity, lipid peroxidation, and mitochondrial dysfunction. Such secondary injury may occur in brain adjacent to the site of initial supposed injury, yielding the potential for unexpected spread of the zone of damage over months post-injury. The damage resulting from this complex cascade of cellular, neurochemical, inflammatory and metabolic events may lead to neuronal loss, dendritic, axonal and synaptic changes, and persistent white matter abnormalities Presently, there are no treatments to counter such adverse outcomes. Thus, developing efficacious therapeutic interventions to protect the brain and promote repair after TBI is a particularly urgent pursuit.

WO 2012/125034 deals with improving neural survival following neurological injury induced by an external force, such as following traumatic brain injury.

An important problem induced by TBI is white matter loss or diffusivity leading to cognitive function disabilities. Reference is made to Cristofori et al. "White and gray matter contributions to executive function recovery after traumatic brain injury" Neurology (2015) vol. 84(14) pages 1394 - 1401. CN103371993 describes the application of omega-3 fatty acids in treatment of traumatic brain injury with an effect both on lesion size as well as white matter (by monitoring the fluorescence intensity of MBP), but still the effect is limited. There is thus still a need for improvement in the treatment of TBI in the art.

Not directed to TBI, WO2015/115885 describes the use of one or more of uridine and cytidine, or salts, phosphates, acyl derivatives or esters thereof in the manufacture of a composition for treating, preventing or reducing the risk of occurrence of white matter lesions, white matter hyperintensities (WMH), Leukoaraiosis or periventricular white matter disease in elderly not suffering from a neurodegenerative disorder, preferably non-demented elderly and elderly not suffering from Alzheimer's Disease.

### Summary of the invention

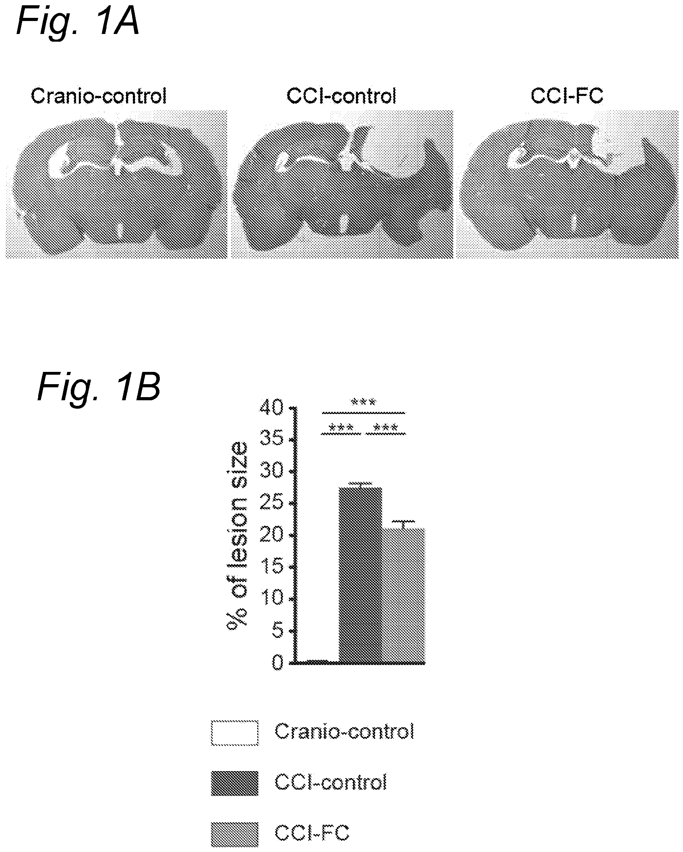

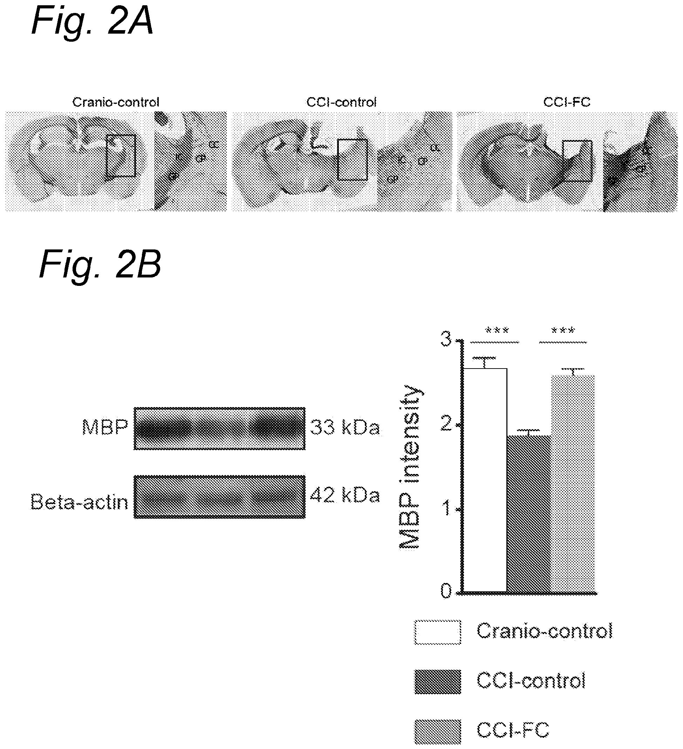

The inventors have observed that after administration of a product comprising (i) one or more of uridine and cytidine, or salts, phosphates, acyl derivatives or esters thereof, (ii) a lipid fraction comprising at least one of docosahexaenoic acid (22:6; DHA), eicosapentaenoic acid (20:5; EPA) and docosapentaenoic acid (22:5; DPA), or esters thereof, in which the lipid fraction comprises less than 2 weight% of α-linolenic acid (ALA), calculated on the weight of all fatty acids, (iii) choline, or salts or esters thereof, (iv) vitamins B6, B9 and B12, and (v) vitamin C, vitamin E and selenium, lesion size is reduced (Figure 1) and white matter is produced (Figure 2) after TBI. The effects of intervention in a TBI mouse model in terms of brain lesion size and white matter (by way of myelin production) were monitored. Intervention showed successful for each of these aspects which were found otherwise compromised in the context of TBI. The results are presented in the experimental part.

Taken from Figure 2 herein, compared to the results obtained with DHA in CN103371993, the present intervention showed greater improvement in myelin production: the CCI-FC levels with the current intervention were almost at the same levels as observed for the craniotomy group (cranio-control), whereas theTBI intervention control (CCI control) group has a significantly lower myelin basic protein (MBP); In contrast, in CN103371993 figures 6D and 6E the average MBP intensity is displayed for a craniotomy group (sham, white bar), a TBI intervention group (RD-CCI, grey bar) and a TBI intervention group treated with a fish oil diet comprising DHA (FOD-CCI, black bar). When the craniotomy group (cranio-control) results of the current invention are set at 1, CCI-FC yielded about 0.9 (vs CCI ctrl 0.7) whereas these values in Figure 6 in CN103371993 are 1, and about 0.5 and 0.65 respectively. Accordingly, the effect obtained with the present intervention shows an almost full restoration of myelin levels compared to control levels, whereas in the FOD-CCI group of CN103371993 there is only slight improvement over the TBI invention group (RD-CCI) and no restoration towards the levels detected in the craniotomy group (sham).

Thus, according to one embodiment, the composition comprises:
(i) one or more of uridine and cytidine, or salts, phosphates, acyl derivatives or esters thereof;
(ii) a lipid fraction comprising at least one of docosahexaenoic acid (22:6; DHA), eicosapentaenoic acid (20:5; EPA) and docosapentaenoic acid (22:5; DPA), or esters thereof, in which the lipid fraction comprises less than 2 weight% of α-linolenic acid (ALA), calculated on the weight of all fatty acids;
(iii) choline, or salts or esters thereof;
(iv) vitamins B6, B9 and B12, and
(v) vitamin C, vitamin E and selenium,
for use in producing myelin in a mammal suffering from or recovering from traumatic brain injury. The composition is preferably administered enterally (preferably orally).

While the individual active ingredients (i) - (v) are suitably administered enterally (preferably orally) in a single composition, they may also be administered in individual dosage units. Hence, the invention further relates to a kit of parts comprising (i) - (v) separately, for use together in producing myelin in a mammal suffering from or recovering from traumatic brain injury.

The ingredients (i), (ii), (iii), (iv) and (v) and further optional ingredients are detailed here below.

In one aspect, associated with the improved myelin production, the invention relates to the composition comprising (i), (ii), (iii), (iv) and (v) and further optional ingredients for use in improving the rate of recovery from TBI.

### List of figures

**Figure 1** shows results of dietary intervention on lesion size and white matter in a mouse model of TBI. A controlled cortical impact (CCI) as model for TBI or a control craniotomy (sham injury) injury were induced in adult male mice. Animals were divided into three experimental groups (craniotomy-control [white bar], CCI-Control [dark-grey] and CCI-FC [light-grey) treated with either a control diet or intervention diet (FC):
   (A). Representative coronal section stained with H&E showing differences in lesion size between the three groups.
   (B) Quantification of the lesion size, showing a significant reduction in size in CCI-FC mice compared to CCI-control mice, at 70 days post-TBI (one-way ANOVA p<0.01; Bonferroni's multiple comparison post-hoc test; ***P <0.001). Lesion size was calculated as (contralateral hemisphere-ipsilateral hemisphere)/contralateral hemisphere, and presented as percentages. Data are presented as means ± SEM of 5 animals per group.
**Figure 2** shows the effect of TBI and treatment with a composition effective in the treatment of TBI on myelin content:
   (A) Representative images of coronal brain histology sections stained with Luxol Fast blue (LFB) for myelin. Note staining differences in the internal capsule (IC), globus pallidus (GP)-external segment, caudate-putamen (CP) and corpus callosum (CC) regions (specific areas marked with rectangles and enlarged). In the CCI-FC and craniotomy-control groups, myelin-stained tracts look continuous, in contrast with a dotted pattern seen in the CCI-control group, both ipsilateral and contralateral to the injury site.
   (B) Myelin-basic protein (MBP) level analyzed by western blotting. The expression of MBP (20 kDa) was significantly higher in the CCI-FC and craniotomy-control groups (one-way ANOVA p<0.001; Bonferroni's multiple comparison post-hoc test ***P<0.001) compared to CCI-control mice. The legend is the same as for Figure 1B.

### Detailed description of the invention

The inventors found that a composition according to the invention is effective in the treatment of TBI and improves the recovery from TBI. In a preferred aspect the composition according to the invention is administered shortly after diagnosis of TBI, preferably starting within 3 months, preferably within 2 months, more preferably within 1 month, more preferably within 2 weeks, most preferably within 1 week after the diagnosis of TBI. In a preferred aspect the administration or use of the composition is administered daily for at least 5 days, preferably at least 7 days, preferably at least 14 days, more preferably at least 1 month, more preferably at least 3 months.

The composition for use of the invention comprises administering the composition at outlined below to a mammal subject in need thereof, preferably a human person in need thereof, suffering from TBI. TBI refers to damage to the brain resulting from external mechanical force. In the context of the invention, TBI can be mild (level of unconsciousness of less than 30 minutes and/or a Glasgow Coma score of 13 - 15) or moderate to severe TBI (level of unconsciousness of more than 30 minutes and/or a Glasgow Coma score of less than 13). TBI can result from falls, firearm wounds, sports accidents, construction accidents and vehicle accidents, among other causes. As it appears, traumatic brain injury is the most prevalent injury of soldiers in combat (e.g. amongst the US troops in Iraq and Afghanistan). Victims of TBI can suffer from a number of physical, cognitive, social, emotional and/or behavioural disorders following injury. The primary impact results in direct neural cell loss predominantly exhibiting necrotic death, which is then generally followed by a wave of secondary injury cascades including excitotoxicity, oxidative stress, mitochondrial dysfunction, blood-brain barrier disruption, and inflammation. According to the diagnostic criteria detailed in the "Diagnostic and Statistical Manual of Mental Disorders (DSM-5) TBI is associated with the unique combination of one or more of the following characteristics: changes in levels of consciousness; memory disturbances; confusion associated with deficits in orientation; neurological signs, such as brain injury observable on neuroimaging, new onset or worsening of seizure disorder, visual field deficits and hemiparesis.

In one aspect the treatment involves improved myelinisation in a patient suffering or recovering from TBI. White matter loss in the context of TBI is different from white matter degeneration associated with neurodegenerative disorders such as Alzheimer's Disease. Treatment preferably involves improved myelin production. White matter disruption is observed in both mild as well as moderate and severe TBI. Intervention with the composition of the invention resulted in enhanced white matter production and improved myelin production. Myelin and white matter production can be monitored using imaging techniques available to skilled practitioners, such as there are Positron Emission Tomography (PET), Single Photon Emission Computerized Tomography (SPECT), Functional Magnetic Resonance Imaging (fMRI) and Diffuse Tensor Imaging (DTI).

In one aspect of the invention, the treatment does not relate to neurogenesis, neuronal survival and neuroinflammation. In another aspect of the invention, mammals suffering from PKU or hyperphenylalaninemia are excluded. In another aspect of the invention, the treatment of white matter disease or periventricular white matter disease or white matter degeneration associated with aging is also excluded.

In one aspect of the present invention, the composition according to the invention may be used as a pharmaceutical product comprising one or more pharmaceutically acceptable carrier materials.

In another aspect of the present invention, the composition according to the invention may be used as a nutritional product, for example as a nutritional supplement, e.g., as an additive to a normal diet, as a fortifier, to add to a normal diet, or as a complete nutrition.

The pharmaceutical product, preferably for enteral application, may be a solid or liquid galenical formulation. Examples of solid galenical formulations are tablets, capsules (e.g. hard or soft shell gelatine capsules), pills, sachets, powders, granules and the like which contain the active ingredient together with conventional galenical carriers. Any conventional carrier material can be utilized. The carrier material can be organic or inorganic inert carrier material suitable for oral administration. Suitable carriers include water, gelatine, gum Arabic, lactose, starch, magnesium stearate, talc, vegetable oils, and the like. Additionally, additives such as flavouring agents, preservatives, stabilizers, emulsifying agents, buffers and the like may be added in accordance with accepted practices of pharmaceutical compounding.

If the composition is a pharmaceutical product, such product may contain the daily dosage in one or more dosage units. The dosage unit may be in a liquid form or in a solid form, wherein in the latter case the daily dosage may be provided by one or more solid dosage units, e.g. in one or more capsules or tablets.

In another aspect of the present invention, the composition according to the invention may be used in a nutritional product comprising fats, proteins, and carbohydrates. It is understood that a nutritional product differs from a pharmaceutical product by the presence of nutrients which provide nutrition to the subject to which the composition is administered, in particular the presence of protein, fat, digestible carbohydrates and dietary fibres. It may further contain ingredients such as minerals, vitamins, organic acids, and flavouring agents. Although the term "nutraceutical product" is often used in literature, it denotes a nutritional product with a pharmaceutical component or pharmaceutical purpose. Hence, the nutritional composition according to the invention may also be used in a nutraceutical product.

The product of the invention is an enteral composition, intended for oral administration. It is preferably administered in liquid form. In one embodiment, the product comprises a lipid fraction and at least one of carbohydrates and proteins, wherein the lipid composition provides between 20 and 50 energy % of the food product. This is based on the assumption that lipid, carbohydrates and protein generate approximately 9, 4 and 4 kcal/g, respectively, the 3 together making up for all caloric contributions of the composition. In one embodiment, the food product is a liquid composition containing between 0.8 and 1.4 kcal per ml.

### DHA/EPA/DPA

The method, product, composition or kit of the invention comprises therapeutically effective amounts of at least one omega-3 long-chain polyunsaturated fatty acid (LC-PUFA; having a chain length of 18 and more carbon atoms) selected from the group consisting of docosahexaenoic acid (22:6, ω-3; DHA), eicosapentaenoic acid (20:5, ω-3; EPA) and docosapentaenoic acid (22:5 ω-3; DPA), preferably at least DHA and/or EPA, more preferably at least DHA, most preferably DHA and EPA. EPA is converted to DPA (ω-3), increasing subsequent conversion of DPA to DHA in the brain. Hence, the present composition preferably contains a significant amount of EPA, so to further stimulate *in vivo* DHA formation.

The DHA, EPA and/or DPA are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition comprises at least DHA in triglyceride form. Suitable ω-3 LCPUFA and/or DHA sources include tuna oil, (other) fish oils, DHA-rich alkyl esters, algae oil, egg yolk, or phospholipids enriched with ω-3 LCPUFA e.g. phosphatidylserine-DHA. Preferably, a product, composition or kit according to the invention comprises fish oil providing the omega-3 LCPUFA(s). Another particularly suitable source for the omega-3 LCPUFA(s) is algae oil.

In terms of daily dosage, the present composition for use preferably comprises the administration of 500 to 5000 mg DHA+EPA+DPA per day. In a preferred embodiment, in terms of daily dosage, the present composition for use preferably comprises the administration of 500 - 5000 mg DHA+EPA per day, more preferably 1000 - 4000 mg per day. DHA is preferably administered in an amount of 400 - 4500 mg per day, more preferably 750 - 3250 mg per day. In addition, EPA is preferably administered in an amount of 100 - 4600 mg per day, more preferably 250 - 3250 mg per day.

In terms of unit dosage, the proportion of DHA+EPA+DPA (preferably DHA+EPA) of the total fatty acids is preferably 5 to 95 weight%, more preferably 10 to 80 weight%, most preferably 15 to 70 weight%. The present composition preferably comprises 5 to 95 weight% DHA based on total fatty acids, preferably 10 to 75 weight% DHA based on total fatty acids, more preferably 10 to 60 weight% DHA based on total fatty acids. The present composition preferably comprises 5 to 95 weight% EPA based on total fatty acids, preferably 10 to 75 weight% EPA, most preferably 15 to 60 weight%, based on total fatty acids.

The ratio of the weights of DHA to EPA is preferably larger than 1, more preferably 2:1 to 10:1, more preferably 3:1 to 8:1. The above-mentioned ratios and amounts take into account and optimise several aspects, including taste (too high LCP levels reduce taste, resulting in a reduced compliance), balance between DHA and precursors thereof to ensure optimal effectiveness while maintaining low-volume formulations.

### ALA/LA

It is preferred that the alpha-linolenic acid [ALA] content of the composition is maintained at low levels. The inventors believe that due to the inflammatory nature of traumatic brain injury, excess supply of highly unsaturated fatty acids increases the risk of further damage to injury tissue due to the effect of peroxidized PUFAs, even though it has been observed that *in vivo* supply of α-linolenic acid is neuroprotective in neurotrauma (King et al. J. Neurosci. (26) 17:4672-4680). It is discovered by the inventors that the ALA concentration is maintained at levels less than 2.0 weight%, more preferably below 1.5 weight%, particularly below 1.0 weight%, calculated on the weight of all fatty acids. In the animal studies attached levels were about 0.8 g per 100 g fatty acids.

Linoleic acid [LA] concentrations can be maintained at normal levels, i.e. between 20 to 30 weight%, although in one embodiment the LA concentration is also significantly reduced to an amount of less than 15 g/100 g fatty acids and preferably even less than 10 weight%. The LA concentrations are preferably at least 1 weight% of the fatty acids. The LA:ALA ratio is preferably in the range of 10: 1 to 7:1.

In one embodiment, the weight ratio ω-3/ ω-6 in the composition of the invention is preferably in the range 0.3 to 7, preferably in the range 1.4:1 to 5.9:1, more preferably in the range 3:1 to 5.5:1, most preferably 3:1 to 5:1, in particular less than 5:1. The amount of ω-6 LCPUFAs is preferably less than 50, preferably 5 to 40, more preferably 8 to 30 weight% of the fatty acids in the formula.

### Medium Chain Triglycerides (MCT)

In one embodiment, the composition, product or kit contains less than 5 weight%, preferably less than 2 weight% of fatty acids of less than 14 carbon atoms. Medium chain fatty acids [MCT] are defined to be linear or branched saturated carboxylic acids having six (C6:0), seven (C7:0), eight (C8:0), nine (C9:0) or ten (C10:0) carbon atoms. The amount of MCTs is preferably lower than 2 weight%, more preferably lower than 1.5 weight%, most preferably lower than 1.0 weight% of the total fatty acids. In one embodiment, the sum of the medium chain fatty acids C6:0 + C7:0 + C8:0 over the sum of C9:0 and C10:0 is less than 2:1, more preferably less than 1.8:1, most preferably less than 1.6:1, particularly less than 1.4:1.

### Uridine or cytidine

The present composition, productor kit involves therapeutically effective amounts of uridine, cytidine and/or an equivalent thereof, including salts, phosphates, acyl derivatives and/or esters. In terms of uridine, the composition preferably comprises at least one uridine or an equivalent thereof selected from the group consisting of uridine (i.e. ribosyl uracil), deoxyuridine (deoxyribosyl uracil), uridine phosphates (UMP, dUMP, UDP, UTP), nucleobase uracil and acylated uridine derivatives. In one embodiment, cytidine, CMP, citicoline (CDP-choline) may also be applied. Preferably, the present composition comprises an uridine phosphate selected from the group consisting of uridine monophosphate (UMP), uridine diphosphate (UDP) and uridine triphosphate (UTP); and/or a cytidine phosphate (CMP, CDP, CTP, preferably CMP). Most preferably the present composition comprises UMP, as UMP is most efficiently being taken up by the body. Preferably at least 50 weight% of the uridine in the present composition is provided by UMP, more preferably at least 75 weight%, most preferably at least 95 weight%. Doses that must be administered are given as UMP. The amount of uracil sources can be calculated taking the molar equivalent to the UMP amount.

The present composition for use preferably comprises the administration of uridine (the cumulative amount of uridine, deoxyuridine, uridine phosphates, nucleobase uracil and acylated uridine derivatives) in an amount of (i) 0.1 to 6 g per day, preferably 0.2 to 3 g per day, more preferably 0.4 to 2 g per day, and/or (ii) 0.1 to 6 g per 100 ml (liquid) composition, preferably 0.2 to 3 g per 100 ml (liquid) composition, more preferably 0.4 to 2 g per 100 ml (liquid) composition. The above amounts also account for any amounts of cytidine, cytidine phosphates and citicoline incorporated in the composition.

Preferably, the present composition comprises uridine phosphate, preferably uridine monophosphate (UMP). The UMP is very efficiently taken up by the body. Hence, inclusion of UMP in the present composition enables a high effectivity at the lowest dosage and/or the administration of a low volume to the subject.

### Choline

The present composition, product or kit involves therapeutically effective amounts of choline, a choline salt and/or choline ester. The choline salt is preferably selected from choline chloride, choline bitartrate, or choline stearate. The choline ester is preferably selected from a phosphatidylcholine and lyso-phosphatidyl choline. The present composition for use preferably comprises the administration of more than 50 mg choline per day, preferably 80 to 3000 mg choline per day, more preferably 100 to 2000 mg choline per day, most preferably 150 to 1000 mg choline per day. The present composition preferably comprises 80 mg to 3000 gram choline per 100 ml of the liquid composition, preferably 100 mg to 2000 mg choline per 100 ml, preferably 200 to 1000 mg choline per 100 ml composition, most preferably 200 mg to 600 mg choline per 100 ml. The above numbers are based on choline, the amounts of choline equivalents or sources can be calculated taking the molar equivalent to choline into account.

### Vitamins

The present composition, product or kit involves therapeutically effective amounts of vitamin B6 (pyridoxine, pyridoxal, or pyridoxamine, or pyridoxine hydrochloride), vitamin B9 (folic acid or folate), and vitamin B12 (cobalamins). Functional equivalents are encompassed within these terms. Good results have been achieved with a combination comprising therapeutically effective amounts of all of vitamin B6, vitamin B12 and vitamin B9.

The vitamins B are to be administered in an effective dose, which dose depends on the type of vitamin B used. As a rule of thumb, a suitable minimum or a maximum dose may be chosen based on known dietary recommendations, for instance as recommended by Institute of Medicine (IOM) of the U.S. National Academy of Sciences or by Scientific Committee on Food (a scientific committee of the EU), the information disclosed herein and optionally a limited amount of routine testing. A minimum dose may be based on the estimated average requirement (EAR), although a lower dose may already be effective. A maximum dose usually does not exceed the tolerable upper intake levels (UL), as recommended by IOM.

The vitamin B6 is preferably present in an amount to provide a daily dosage in the range of 0.1 to 100 mg, in particular in the range of 0.5 to 25 mg, more in particular in the range of 0.5 to 5 mg. The present composition preferably comprises 0.1 - 100 mg vitamin B6 per 100 g (liquid) product, more preferably 0.5 - 5 mg vitamin B6 per 100 g (liquid) product, more preferably 0.5 - 5 mg vitamin B6 per 100 g (liquid) product.

The vitamin B12 is preferably present in an amount to provide a daily dosage in the range of 0.5 to 100 µg, in particular in the range of 1 to 10 µg, more in particular in the range of 1.5 to 5 µg. The present composition preferably comprises 0.5-100 µg vitamin B12 per 100 g (liquid) product, more preferably 1-10 µg vitamin B12 per 100 g (liquid) product, more preferably 1.5-5 µg vitamin B12 per 100 g (liquid) product. The term 'vitamin B12' incorporates all cobalbumin equivalents known in the art.

The vitamin B9 (folic acid) is preferably present in an amount to provide a daily dosage in the range of 50 to 5000 µg, in particular in the range of 100 to 1000 µg, more in particular in the range of 200 to 800 µg. The present composition preferably comprises 50-5000 µg folic acid per 100 g (liquid) product, more preferably 100-1000 µg folic acid per 100 g (liquid) product, more preferably 200 - 800 µg folic acid per 100 g (liquid) product. Folates include folic acid, folinic acid, methylated, methenylated and formylated forms of folates, their salts or esters, as well as their derivatives with one or more glutamic acid, and all in either reduced or oxidized form.

### Phospholipids

It is preferred to incorporate at least one phospholipid in the composition. The term "phospholipid" excludes PC that is already accounted for in the choline fraction. The present composition preferably comprises at least one phospholipid in an amount of 0.01 to 1 gram per 100 ml, more preferably between 0.05 and 0.5 gram per 100 ml, most preferably 80 to 600 mg per 100 ml. The at least one phospholipid is preferably provided for using lecithin, more preferaly soy lecithin.

### Vitamins C, E, selenium

The present composition, product or kit involves therapeutically effective amounts of the antioxidants vitamin C, vitamin E and selenium.

Vitamin C, or a functional equivalent thereof, may be present in an amount to provide a daily dosage in the range of 20 to 2000 mg, in particular in the range of 30 to 500 mg, more in particular in the range of 75 to150 mg. In one embodiment, vitamin C , or a functional equivalent thereof, is present in an amount in the range of 20 to 2000 mg, in particular in the range of 30 to 500 mg, more in particular in the range of 75 to150 mg per 100 ml of the composition.

Tocopherol and/or an equivalent thereof (i.e. a compound having vitamin E activity) may be present in an amount to provide a daily dosage in the range of 10 to 300 mg, in particular in the range of 30 to 200 mg, more in particular in the range of 35 to100 mg, to prevent oxidative damage to the injury site resulting from dietary PUFA. In one embodiment, tocopherol and/or equivalent is present in an amount in the range of 10 to 300 mg, in particular in the range of 30 to 200 mg, more in particular in the range of 35 to100 mg per 100 ml of the composition. The term "tocopherol and/or an equivalent there of", as used in this description, comprises tocopherols, tocotrienols, pharmaceutical and/or nutritional acceptable derivatives thereof and any combination thereof. The above numbers are based on tocopherol equivalents, recognized in the art.

The present composition also contains selenium. The antioxidant activity of selenium advantageously prevents and/or inhibits damages to the brain areas. Preferably the present composition for use provides the administration of a composition comprising 0.01 and 5 mg selenium per 100 ml liquid product, preferably 0.02 and 0.1 mg selenium per 100 ml liquid product. The amount of selenium administered per day is preferably more than 0.01 mg, more preferably 0.01 to 0.5 mg.

The composition, product or kit may further comprise proteinaceous material. Should a protein fraction be included, the protein fraction comprises intact proteins, peptides as may be obtained by hydrolyses of intact proteins and by syntheses, derivatives of peptides comprising more than 80 weight% amino acids. In the context of the invention, nitrogen from nucleosides material and choline will not be calculated as being protein.

It is preferred that the amount of taurine (including taurine salts) is less than 0.1 g, preferably less than 0.05 g per daily dose. Additionally or alternatively, it is preferred that the amount of taurine (including taurine salts) is less than 5 mg, more preferably less than 2.5 g per 100 g composition. In one embodiment, the composition comprises less than 25 mg, more preferably less than 20 mg, most preferably less than 15 mg cysteine and taurine per 100 ml of the (liquid) composition. In one embodiment, the composition comprises less than 25 mg, more preferably less than 20 mg, most preferably less than 15 mg cysteine per 100 ml of the (liquid) composition. It is preferred that the protein fraction comprises more than 70 weight% of casein or caseinates, or hydolysates thereof, and more preferably 80 weight% or more, because caseins comprise relatively low amounts of cysteine compared to other protein sources. It is further preferred to heat the liquid composition in order to oxidize the cysteine molecules present in the protein. This impairs biological availability of any residual cysteine as present in the formula. A preferred heat treatment involves sterilization. It is preferred to maintain the temperature remains below 135 °C, preferably less than 132 °C combined with a sufficient long time to have the cysteine oxidized, i.e. more than 30 seconds, preferably more than 40 seconds.

In one embodiment, it is preferred that the composition has a protein content of less than 15 en%, more preferably less than 10 en%, most preferably less than 5 en% of the total energy content of the composition. The energy percentages of the components are calculated using the calculation factors 9 kcal per g lipid, 4 kcal per g protein or g digestible carbohydrates, 2 kcal per g dietary fibers and zero kcal for the other components in the composition. In one embodiment, it is preferred that the composition comprises less than 0.5 to 10 g protein per 100 ml, more preferably less than 1 to 6 gram protein per 100 ml, most preferably 2 to 6 gram protein/100 ml.

It has been found that enhanced levels of manganese and molybdenum are not necessary in the composition for use according to the invention to achieve a beneficial effect on TBI. The amount of manganese consumed/administered in the composition for use of the invention is preferably less than 300 µg per 100 ml, preferably less than 250 µg per 100 ml, more preferably less than 100 µg per 100 ml, in particular less than 60 µg per 100 ml. In one embodiment, the amount of manganese administered per day is preferably less than 100 µg, more preferably less than 50 µg. In one embodiment, 100 ml liquid composition according to the invention comprises less than 0.05 mg molybdenum, preferably less than 0.025 mg molybdenum.

In one embodiment, the composition according to the invention comprises per daily dosage or per 125 ml of liquid (preferably water) :
100 - 600 mg, preferably 200 - 500 mg, more preferably about 300 mg EPA;
900 - 1500 mg, preferably 950 - 1300 mg, more preferably about 1200 mg DHA;
50 - 600 mg, preferably 60 - 200 mg, more preferably about 106 mg phospholipids;
200 - 600 mg, preferably 300 - 500 mg, more preferably about 400 mg choline;
400 - 800 mg, preferably 500 - 700 mg, more preferably about 625 mg UMP (uridine monophosphate);
20 - 60 mg, preferably 30 - 50 mg, more preferably about 40 mg vitamin E (alpha-TE);
60 - 100 mg, preferably 60 - 90 mg, more preferably about 80 mg vitamin C;
40 - 80 µg, preferably 45 - 65 µg, more preferably about 60 µg selenium;
1 - 5 µg, preferably 2 - 4 µg, more preferably about 3 µg vitamin B12;
0.5 - 3 mg, preferably 0.5 - 2 mg, more preferably about 1 mg vitamin B6; and
200 - 600 µg, preferably 300 - 500 µg, more preferably about 400 µg folic acid.

The compositions as described above can be used as a nutritional therapy, nutritional support, as a medical food, as a food for special medical purposes or as a nutritional supplement. Such product can be consumed at one, two or three servings of 125 mL per day during recovery and/or rehabilitation from TBI.

### EXAMPLES

For a more complete understanding of the present disclosure, reference is now made to the following examples taken in conjunction with the accompanying drawings.

### Example 1 - Exploration of the neuroprotective effects in a mouse model of TBI

The effects of the composition comprising a diet according to the invention comprising and comprising ingredient as set out in table 1 was assessed in an experimental model of traumatic brain injury. Table 1 sets out the ingredients of the control and FC diet.

Adult 10-12 week-old male C57BL/6 mice, weighing 22-27 g (Charles River Laboratories, Harlow, UK), were used. Mice were housed in groups of four in standard cages provided with enrichment objects, in a 12 h light/dark cycle, and given diet and water ad libitum. Food intake and body weight were monitored daily. All animal procedures were approved by the Animal Welfare and Ethical Review Body, at Queen Mary University of London and the UK Home Office, in accordance with the EU Directive 2010/63/EU.

A controlled cortical impact (CCI) TBI model and a craniotomy only (control) model were used in this study. Briefly, after a 1-week acclimatisation period, mice were anaesthetized using a mixture of ketamine (50 mg/Kg) and medetomidine (0.5 mg/Kg) in sterile saline, administered intraperitoneally (i.p.). Mice were placed in a stereotaxic frame and a midline longitudinal incision was performed to expose the skull. A right lateral craniotomy was carried out using a pneumatic drill, 2.0 mm behind Bregma and 2.5 mm lateral to the midline. CCI injury was induced using the following settings: a 3 mm impactor tip with a speed of 3 m/s, a depth of 2.2 mm and a dwell time of 100 ms, applied using the PCI3000 Precision Cortical Impactor^{™} (Hatteras Instruments, Inc., US). A control group underwent craniotomy only. After injury, the skull flap was placed back and the skin was sutured. Mice were allowed to recover in an incubator (37°C) until they were fully awake and active. Buprenorphine (0.05 mg/kg) administered subcutaneously (s.c.) was used preoperatively for pre-emptive analgesia and post-operatively every 12 h for 3 days post-TBI.

Following the injury, the CCI mice were randomized into two groups and fed daily with either a fresh control diet ('CCI-Control'; n=10), or with a multi-nutrient intervention diet ('CCI-FC'; n=10) for 70 days. The craniotomy group of mice were daily fed with control diet (Craniotomy; n=10). The diets were formulated by Nutricia Research, Nutricia Advanced Medical Nutrition (Utrecht, The Netherlands) and manufactured and pelleted by Ssniff (Soest, Germany). Diets were stored at - 20 °C until use, to prevent lipid oxidation, and fresh diet was given to the animals daily. No significant differences were observed in the mean daily food intake and body weight gain (between groups), throughout the experiment.

**Table 1: Nutritional composition of the control and intervention diet (FC) in g/100g diet**

| | **g / 100 g diet** | **g / 100 g diet** |
|---|---|---|
| | **Control** | **FC** |
| Cornstarch, pre-gelatinized | 35.59 | 32.51 |
| Caseine | 14.00 | 14.00 |
| Maltodextrin, 10 DE | 15.50 | 15.50 |
| Sucrose | 10.00 | 10.00 |
| Dextrose | 10.00 | 10.00 |
| Cellulose powder | 5.00 | 5.00 |
| Mineral & trace element premix (AIN-93M-MX) | 3.50 | 3.50 |
| Vitamin mix (AIN-93-VX) | 1.00 | 1.00 |
| | | |

| **Fats:** | | |
|---|---|---|
| Soy oil | 1.900 | |
| Coconut oil | 0.900 | 0.100 |
| Corn oil | 2.200 | 1.700 |
| Sup. Ref. tuna fish oil (HiDHA 25N Food) | | 3.000 |
| Refined fish oil (EPA) | | 0.200 |

| **Additions:** | | |
|---|---|---|
| L-cystine | 0.180 | 0.180 |
| Choline chloride (50%. 0.434 g / g) | 0.230 | 0.922 |
| Tert-butylhydroquinone | 0.0008 | 0.0008 |
| | | |
| Soy lecithin (Emulpur) | | 0.7547 |
| UMP disodium (24%H2O) | | 1.0000 |
| Ascorbic acid (100% pure) | | 0.1600 |
| Vit. E (Tocopherol acetate, 50 %) | | 0.4650 |
| Vit. B6 (Pyridoxin hydrochloride, 82 %) | | 0.0033 |
| Folic acid (100%) | | 0.0006 |
| Vit. B12 (Cyanocobalamin 0.1 %) | | 0.0035 |
| Na selenite • 5 H₂O | | 0.0003 |
| | | |
| Total | 100.0 | 100.0 |
| Energy (kcal/100g chow) | 377.7 | 365.4 |

At day 70 post-TBI, 5 animals from each group were deeply anaesthetized with sodium pentobarbital (50 mg/kg, i.p.; Sagatal, Rhone Merieux, Harlow, UK), and received a transcardiac perfusion with phosphate-buffered saline (PBS; 0.01 M, pH 7.4), followed by 4% paraformaldehyde (PFA) in phosphate buffer (0.1 M, pH 7.4, 4°C). The brains were dissected out and brain tissue blocks were paraffin-fixed for histology and immunohistochemistry (IHC).

All tissue staining was performed between bregma - 1.28 and bregma -2.34, where the lesion was located. Briefly, 7 µm sections were deparaffinised and hydrated through a series of xylene and ethanol baths. Sections were subjected to antigen retrieval with 10 mM citrate buffer (pH 6.0) for 30 min at 80°C and allowed to cool at room temperature for 30 min. Then, the tissue was blocked with 5% normal donkey serum in 0.2% Triton X-100 in PBS for an hour, followed by three PBS washes and overnight staining with a primary antibody. The secondary antibodies were Alexa 488 or Alexa 555 (Molecular Probes, Leiden, The Netherlands; 1 :200), and Hoechst 33342 stain (Sigma, UK; 1 µg/ml PBS) was used to visualize nuclei. Slides were mounted and cover-slipped using Vectashield fluorescent mounting medium (H-1000; Vector Laboratories, Burlingame, CA).

### Western blotting

Tissue from 5 animals from each group (CCI or craniotomy) was used for western blot analysis. After decapitation on day 70, brains were quickly removed and a cube of the right hemisphere around the lesion was dissected using a sagittal brain slicer matrix. The tissue was snap frozen and stored at -80_{°}C until analysis. Brain samples were prepared in RIPA lysis buffer (Sigma-Aldrich) complete with Protease Inhibitor Cocktail (Sigma-Aldrich) and sonicated. Samples were then centrifuged at 10,000 g for 10 min at 4°C and the supernatant was taken. Protein concentrations were determined using the Bradford protein assay. Equal amounts of protein (50 µg) from each sample were mixed with NuPAGE^{®} LDS sample buffer (Thermo Fisher Scientific) and dithiothreitol (DTT) and boiled at 95°C for 10 min before loading. Proteins were separated using Mini-Protean TGX Gels, 10% (Biorad, UK) and electro-transferred onto polyvinylidene difluoride membranes (Biotrace). Membranes were blocked in 5% non-fat dry milk in Tris-buffered saline (pH 7.4), with 0.1% Tween-20 (Tris-buffered saline-Tween) for 1 h at room temperature. The primary antibodies used was: mouse anti-MBP (1:1,000; Abeam), diluted in 5% bovine serum albumin solution, and membranes were incubated overnight at 4_{°}C. The primary antibody was removed and the blots were washed in Tris-buffered saline-Tween and incubated for 1 h at room temperature in horseradish peroxidase-conjugated secondary antibodies (1:10,000; Jackson Immunoresearch). Reactive proteins were visualized using an enhanced chemiluminescence reagent (VWR International). Optical density was determined using Imaged software (National Institutes of Health). All membranes were also incubated with a mouse polyclonal antibody for β-actin (1:4,000; Merck Millipore). Protein level was expressed as relative optical density, representing the optical density of the band revealed by the primary antibody, divided by the optical density of β-actin within the same lane.

### Lesion size

For calculation of the lesion size, sections of 7 µm, 200 µm apart and spanning the entire rostro-caudal extent of the injured cortex were stained with haematoxylin and eosin. The size of the lesion was measured with imaged software (National Institutes of Health, Bethesda, MD, USA). The lesion size was calculated using the following equation: the contralateral (non-lesioned) hemisphere size minus the injured hemisphere size and divided by the contralateral hemisphere size,. The results are expressed as a percentage of hemispheric tissue.

Analysis of the lesion size in the CCI model showed that at 70 dpi there was a significant loss of brain tissue, with almost total loss of the ipsilateral hippocampus. FC supplementation led to a significantly decreased lesion size (Fig. 1A and B).

### White matter - Myelin

A subset of representative randomly selected sections across the whole lesion was used for Luxol Fast Blue (Sigma, UK) myelin staining. Luxol Fast Blue staining (LFB) of the brain sections from animals following injury showed myelin disruption after CCI, particularly apparent within the caudate-putamen and internal capsule. Qualitative observations indicated preserved patterns of myelin in these regions in CCI-FC and craniotomy-control mice (Fig. 2A) with myelin-stained tracts that look continuous, whereas CCI-control diet animals showed severely disrupted patterns.

Tissue myelin basic protein (MBP) measurements using Western Blotting showed a statistically significant decrease in expression levels in CCI mice fed with the control diet, with the levels being fully restored after FC-treatment (Fig. 2B).Overall, these results indicate that the FC diet restores the TBI associated demyelinisation process and protects the white matter post-TBI.

### Example 2: Liquid product containing per 125 ml serving:

| | |
|---|---|
| Energy, kcal 125 | Calcium, mg 100 |
| Protein, g 3.8 | Phosphorus, mg 87.5 |
| Carbohydrate, g 16.5 | Magnesium, mg 25.0 |
| Fat, g 4.9 | Iron, mg 2 |
| EPA, mg 300 | Zinc, mg 1.5 |
| DHA, mg 1200 | Iodine, µg 16.3 |
| Phospholipids, mg 106 | Manganese, mg 0.41 |
| Choline, mg 400 | Copper, µg 225 |
| UMP (uridine monophosphate), mg 625 | Molybdenum, µg 12.5 |
| Vitamin E (alpha-TE), mg 40 | Chromium, µg 8.4 |
| Vitamin C, mg 80 | Vitamin A, µg 200 |
| Selenium, µg 60 | Thiamin (B1), mg 0.19 |
| Vitamin B12, µg 3 | Riboflavin (B2), mg 0.20 |
| Vitamin B6, mg 1 | Niacin (B3), mg NE 2.25 |
| Folic acid, µg 400 | Pantothenic acid (B5), mg 0.66 |
| Sodium, mg 125 | Vitamin D, µg 0.88 |
| Potassium, mg 187.5 | Biotin, µg 5.0 |
| Chloride, mg 156.3 | Vitamin K, µg 6.6 |

| | |
|---|---|
| Abbreviations: EPA, eicosapentaenoic acid; DHA, docosahexaenoic acid; TE, tocopherol equivalents; NE, niacin equivalents. | |

## Claims

1. A composition for use in producing myelin in a mammal suffering from or recovering from traumatic brain injury, comprising enterally administering to the subject a composition comprising therapeutically effective amounts of:
(i) one or more of uridine, cytidine, or salts, phosphates, acyl derivatives or esters thereof;
(ii) a lipid fraction comprising at least one of docosahexaenoic acid (22:6; DHA), eicosapentaenoic acid (20:5; EPA) and docosapentaenoic acid (22:5; DPA), preferably at least DHA, more preferably DHA and EPA,
wherein the lipid fraction comprises less than 2 weight% of α-linolenic acid (ALA), calculated on the weight of all fatty acids;
(iii) choline, or salts or esters thereof;
(iv) vitamins B6, B9 and B12; and
(v) vitamin C, vitamin E and selenium.

2. The composition for use according to claim 1, wherein the lipid fraction comprises medium chain fatty acids, wherein the sum of medium chain fatty acids C6:0 + C7:0 + C8:0 over the sum of C9:0 and C10:0 is less than 2:1.

3. The composition for use according to claim 1 or 2, wherein the lipid fraction comprises less than 2 weight% fatty acids of less than 14 carbon atoms, based on total fatty acids.

4. The composition for use according to any one of the preceding claims, wherein the composition further comprises linoleic acid (LA) in an amount of less than 15 g/100 g fatty acids.

5. The composition for use according to any one of the preceding claims, comprising 500 - 5000 mg DHA+EPA per day.

6. The composition for use according to any one of the preceding claims wherein the composition comprises 100 to 6000 mg per day of one or more of uridine, cytidine, or salts, phosphates or esters thereof, calculated as uridine and cytidine, preferably 100 - 6000 mg per day of uridine or salts, phosphates or esters thereof, calculated as uridine.

7. The composition for use according to any one of the preceding claims, wherein the composition comprises more than 50 mg per day of choline, or salts or esters thereof, calculated as choline.

8. The composition for use according to any one of the preceding claims, wherein the composition further comprises at least one phospholipid.

9. The composition for use according to any one of the preceding claims, wherein the composition comprises, per 100 ml of liquid:
100 - 500 mg EPA,
1000 - 1500 mg DHA,
80 - 600 mg phospholipids,
200 - 600 mg choline,
400 - 800 mg uridine monophosphate (UMP),
20 - 60 mg alpha-TE (vitamin E),
60 - 100 mg vitamin C,
40 - 80 µg selenium,
1 - 5 µg vitamin B12,
0.5 - 2 mg vitamin B6, and
200 - 600 µg folic acid.

10. The composition for use according to any one of the preceding claims, wherein the composition is administered to said mammal starting within 1 month from TBI being diagnozed.

11. The composition for use according to any one of the preceding claims, for use in improving rate of discovery from traumatic brain injury.

12. A kit for use in producing myelin in a mammal suffering from or recovering from traumatic brain injury, said kit comprising containers with therapeutically effective amounts of:
(i) one or more of uridine, cytidine, or salts, phosphates, acyl derivatives or esters thereof;
(ii) a lipid fraction comprising at least one of docosahexaenoic acid (22:6; DHA), eicosapentaenoic acid (20:5; EPA) and docosapentaenoic acid (22:5; DPA), wherein the lipid fraction comprises less than 2 weight% of α-linolenic acid (ALA), calculated on the weight of all fatty acids;
(iii) choline, or salts or esters thereof;
(iv) vitamins B6, B9 and B12; and
(v) vitamin C, vitamin E and selenium.

## Patentansprüche

1. Zusammensetzung zur Verwendung bei der Herstellung von Myelin in einem Säugetier, das an einer traumatischen Hirnverletzung leidet oder sich davon erholt, umfassend die enterale Verabreichung einer Zusammensetzung an das Subjekt, umfassend die therapeutisch wirksame Mengen von:
(i) einem oder mehreren von Uridin, Cytidin oder Salzen, Phosphaten, Acylderivaten oder Estern davon;
(ii) einer Lipidfraktion, die mindestens eines von Docosahexaensäure (22:6; DHA), Eicosapentaensäure (20:5; EPA) und Docosapentaensäure (22:5; DPA), bevorzugt mindestens DHA, stärker bevorzugt DHA und EPA,
wobei die Lipidfraktion weniger als 2 Gew.-% α-Linolensäure (ALA), berechnet auf das Gewicht aller Fettsäuren, enthält;
(iii) Cholin oder Salze oder Ester davon;
(iv) die Vitamine B6, B9 und B12; und
(v) Vitamin C, Vitamin E und Selen.

2. Zusammensetzung zur Verwendung nach Anspruch 1, wobei die Lipidfraktion mittelkettige Fettsäuren umfasst, wobei die Summe der mittelkettigen Fettsäuren C6:0 + C7:0 + C8:0 über die Summe von C9:0 und C10:0 weniger als 2:1 beträgt.

3. Zusammensetzung zur Verwendung nach Anspruch 1 oder 2, wobei die Lipidfraktion weniger als 2 Gew.-% Fettsäuren mit weniger als 14 Kohlenstoffatomen, bezogen auf die gesamten Fettsäuren, umfasst.

4. Zusammensetzung zur Verwendung nach einem der vorhergehenden Ansprüche, wobei die Zusammensetzung ferner Linolsäure (LA) in einer Menge von weniger als 15 g/100 g Fettsäuren enthält.

5. Zusammensetzung zur Verwendung nach einem der vorhergehenden Ansprüche, die 500 - 5000 mg DHA+EPA pro Tag enthält.

6. Zusammensetzung zur Verwendung nach einem der vorhergehenden Ansprüche, wobei die Zusammensetzung 100 bis 6000 mg pro Tag von einem oder mehreren von Uridin, Cytidin oder Salzen, Phosphaten oder Estern davon, berechnet als Uridin und Cytidin, bevorzugt 100 - 6000 mg pro Tag von Uridin oder Salzen, Phosphaten oder Estern davon, berechnet als Uridin, enthält.

7. Zusammensetzung zur Verwendung nach einem der vorhergehenden Ansprüche, wobei die Zusammensetzung mehr als 50 mg pro Tag an Cholin oder Salzen oder Estern davon, berechnet als Cholin, enthält.

8. Zusammensetzung zur Verwendung nach einem der vorhergehenden Ansprüche, wobei die Zusammensetzung ferner mindestens ein Phospholipid enthält.

9. Zusammensetzung zur Verwendung nach einem der vorhergehenden Ansprüche, wobei die Zusammensetzung pro 100 ml Flüssigkeit umfasst:
100 - 500 mg EPA,
1000 - 1500 mg DHA,
80 - 600 mg Phospholipide,
200 - 600 mg Cholin,
400 - 800 mg Uridinmonophosphat (UMP),
20 - 60 mg alpha-TE (Vitamin E),
60 - 100 mg Vitamin C,
40 - 80 µg Selen,
1 - 5 µg Vitamin B12,
0,5 - 2 mg Vitamin B6, und
200 - 600 µg Folsäure.

10. Zusammensetzung zur Verwendung nach einem der vorhergehenden Ansprüche, wobei die Zusammensetzung dem Säugetier innerhalb eines Monats nach der Diagnose von TBI verabreicht wird.

11. Zusammensetzung zur Verwendung nach einem der vorhergehenden Ansprüche, zur Verwendung bei der Verbesserung der Entdeckungsrate von traumatischen Hirnverletzungen.

12. Kit zur Verwendung bei der Herstellung von Myelin in einem Säugetier, das an einer traumatischen Hirnverletzung leidet oder sich davon erholt, wobei das Kit Behälter mit therapeutisch wirksamen Mengen umfasst von:
(i) einem oder mehreren von Uridin, Cytidin oder Salzen, Phosphaten, Acylderivaten oder Estern davon;
(ii) eine Lipidfraktion, die mindestens eines von Docosahexaensäure (22:6; DHA), Eicosapentaensäure (20:5; EPA) und Docosapentaensäure (22:5; DPA) umfasst, wobei die Lipidfraktion weniger als 2 Gew.-% α-Linolensäure (ALA) umfasst, berechnet auf das Gewicht aller Fettsäuren;
(iii) Cholin oder Salze oder Ester davon;
(iv) Vitamine B6, B9 und B12; und
(v) Vitamin C, Vitamin E und Selen.

## Revendications

1. - Composition pour utilisation dans la production de myéline dans un mammifère souffrant ou se remettant d'une lésion cérébrale traumatique, comprenant l'administration entérale au sujet d'une composition comprenant des quantités thérapeutiquement efficaces de :
(i) un ou plusieurs parmi l'uridine, la cytidine ou les sels, phosphates, dérivés acylés ou esters de celles-ci ;
(ii) une fraction lipidique comprenant au moins l'un parmi l'acide docosahexaénoïque (22:6 ; DHA), l'acide eicosapentaénoïque (20:5 ; EPA) et l'acide docosapentaénoïque (22:5 ; DPA), de préférence au moins le DHA, de façon davantage préférée le DHA et l'EPA,
la fraction lipidique comprenant moins de 2 % en poids d'acide α-linolénique (ALA), calculés sur le poids de tous les acides gras ;
(iii) choline, ou sels ou esters de celle-ci ;
(iv) vitamines B6, B9 et B12 ; et
(v) vitamine C, vitamine E et sélénium.

2. - Composition pour utilisation selon la revendication 1, dans laquelle la fraction lipidique comprend des acides gras à chaîne moyenne, la somme des acides gras à chaîne moyenne C6:0 + C7: 0 + C8:0 sur la somme de C9:0 et C10:0 étant inférieure à 2:1.

3. - Composition pour utilisation selon l'une des revendications 1 ou 2, dans laquelle la fraction lipidique comprend moins de 2 % en poids d'acides gras de moins de 14 atomes de carbone, sur la base des acides gras totaux.

4. - Composition pour utilisation selon l'une quelconque des revendications précédentes, dans laquelle la composition comprend en outre de l'acide linoléique (LA) en une quantité inférieure à 15 g/100 g d'acides gras.

5. - Composition pour utilisation selon l'une quelconque des revendications précédentes, comprenant 500 à 5000 mg de DHA+EPA par jour.

6. - Composition pour utilisation selon l'une quelconque des revendications précédentes, dans laquelle la composition comprend 100 à 6000 mg par jour d'un ou plusieurs parmi l'uridine, la cytidine ou les sels, phosphates ou esters de celles-ci, calculés en tant qu'uridine et cytidine, de préférence 100 à 6000 mg par jour d'uridine ou de sels, phosphates ou esters de celle-ci, calculés en tant qu'uridine.

7. - Composition pour utilisation selon l'une quelconque des revendications précédentes, dans laquelle la composition comprend plus de 50 mg par jour de choline, ou de sels ou esters de celle-ci, calculés en tant que choline.

8. - Composition pour utilisation selon l'une quelconque des revendications précédentes, dans laquelle la composition comprend en outre au moins un phospholipide.

9. - Composition pour utilisation selon l'une quelconque des revendications précédentes, dans laquelle la composition comprend, pour 100 ml de liquide : 100 à 500 mg d'EPA,
1000 à 1500 mg de DHA,
80 à 600 mg de phospholipides,
200 à 600 mg de choline,
400 à 800 mg d'uridine monophosphate (UMP),
20 à 60 mg d'alpha-TE (vitamine E),
60 à 100 mg de vitamine C,
40 à 80 µg de sélénium,
1 à 5 µg de vitamine B12,
0,5 à 2 mg de vitamine B6, et
200 à 600 µg d'acide folique.

10. - Composition pour utilisation selon l'une quelconque des revendications précédentes, dans laquelle la composition est administrée audit mammifère en commençant dans 1 mois à compter du moment où la lésion cérébrale traumatique a été diagnostiquée.

11. - Composition pour utilisation selon l'une quelconque des revendications précédentes, pour utilisation dans l'amélioration du taux de découverte des lésions cérébrales traumatiques.

12. - Kit pour utilisation dans la production de myéline dans un mammifère souffrant ou se remettant d'une lésion cérébrale traumatique, ledit kit comprenant des récipients contenant des quantités thérapeutiquement efficaces de :
(i) un ou plusieurs parmi l'uridine, la cytidine ou les sels, phosphates, dérivés acylés ou esters de celles-ci ;
(ii) une fraction lipidique comprenant au moins l'un parmi l'acide docosahexaénoïque (22:6 ; DHA), l'acide eicosapentaénoïque (20:5 ; EPA) et l'acide docosapentaénoïque (22:5 ; DPA), la fraction lipidique comprenant moins de 2 % en poids d'acide α-linolénique (ALA), calculés sur le poids de tous les acides gras ;
(iii) choline, ou sels ou esters de celle-ci ;
(iv) vitamines B6, B9 et B12 ; et
(v) vitamine C, vitamine E et sélénium.
